# EUROPEAN PATENT APPLICATION

(11) **EP 3 025 752 A1**
(43) Date of publication of application: **01.06.2016**
(21) Application number: 15196164.6
(22) Date of filing: 25.11.2015
(51) Int. Cl.: A61M 25/00

(54) **CATHETER**

(30) Priority: 27.11.2014 JP 2014239529
(71) Applicant: ASAHI INTECC CO., LTD., Nagoya-shi, Aichi 463-0024 (JP)
(72) Inventor: Kanazawa, Yuya, Nagoya-shi, Aichi 463-0024 (JP)
(74) Representative: Winter, Brandl, Fürniss, Hübner, Röss, Kaiser, Polte - Partnerschaft mbB

(57) **Abstract**

A catheter including a hollow cylindrical main body formed of metallic wire and a hollow distal end tip formed at a distal end of the cylindrical main body by melting the metallic wire of the cylindrical main body.

## Description

### BACKGROUND OF THE INVENTION

### Field of the Invention

The present invention relates to a catheter, and more particularly to a catheter suitable for passing a hard lesion and the like.

### Description of the Related Art

Conventionally, a catheter is used for treating the inside of a body lumen of a blood vessel and the like. For example, when an occluded portion, a stenosed portion or the like is generated in a lumen of a blood vessel and the like, a catheter is passed through the occluded portion or the stenosed portion to recover their functions. Such a catheter is required to have flexibility allowing it to move forward to a target region through a lumen of a blood vessel and the like. In addition, the distal end of the catheter is required to be excellent in passing performance into a occluded portion, a stenosed portion, and the like, and to have an enough rigidity, for example, so that the catheter can pass through the inside of the occluded portion, the stenosed portion, and the like.

As a catheter excellent in both flexibility of a main body and rigidity of a distal end, there is known a catheter with a metallic distal end tip. For example, Patent Literature 1 discloses an intraosseous catheter that is provided, at the distal end of a flexible cylindrical main body thereof, with a cylindrical metallic distal end tip and is inserted into a medullary cavity of a bone.

However, when the catheter is inserted into a hard lesion such as a occluded portion, a stenosed portion, and the like, the catheter is rotated or moved forward or backward in a state where the distal end of the catheter is in contact with the occluded portion or the stenosed portion and is hard to move. Thus, a torque force is applied on the distal end of the catheter. In the case of the conventional catheter with a distal end tip attached to the main body as a separate member, when the torque force is large, a rupture occurs easily at a border between the main body and the distal end tip.

### Prior Art Documents

### Patent Literature 1

Japanese Patent Application Laid-open No. 2007-244492

### SUMMARY OF THE INVENTION

The object of the present invention is to provide a catheter that is excellent in flexibility and passing performance into an occluded portion, a stenosed portion, and the like, and is capable of effectively preventing a rupture at a border between a distal end tip and a main body.

A catheter of the present invention includes a hollow cylindrical main body that is formed of at least one metallic wire, and a hollow distal end tip that is formed at a distal end of the cylindrical main body by melting a distal end of the metallic wire of the cylindrical main body.

It is preferable that the cylindrical main body is formed by winding the metallic wire into a helical coil structure around a central axis in a longitudinal direction.

It is preferable that the cylindrical main body is formed by winding a plurality of metallic wires into a helical coil structure around a central axis in the longitudinal direction.

The catheter of the present invention includes a hollow cylindrical main body that is formed of metallic wire, and a hollow distal end tip that is formed at a distal end of the cylindrical main body by melting the metallic wire of the cylindrical main body. Thus, the catheter is excellent in integrity between the distal end tip and the cylindrical main body, which effectively prevents a rupture at a border between the distal end tip and the cylindrical main body. Moreover, the flexibility of the distal end of the catheter is not deteriorated.

When the cylindrical main body is formed by winding metallic wire into a helical coil structure around a central axis in a longitudinal direction, the cylindrical main body has excellent flexibility. In this case, a difference in flexibility between the metallic tip and the cylindrical main body is increased. Therefore, in the conventional catheter constituted by the metallic tip and the cylindrical main body as separate members, a rupture easily occurs between the metallic tip and the cylindrical main body. By contrast, in the present invention, the integrity between the metallic tip and the cylindrical main body is excellent, thereby preventing a rupture therebetween more effectively.

When the cylindrical main body is formed by winding a plurality of metallic wires into a helical coil structure around a central axis in a longitudinal direction, the flexibility of the cylindrical main body is further improved. Also in this case, the integrity between the metallic tip and the cylindrical main body is excellent, thereby preventing a rupture therebetween more effectively. In addition, the number of ends of the metallic wire that are positioned at the most distal end of the cylindrical main body is plural, and the number of the metallic wires joined by melting during formation of the distal end tip is increased. Therefore, the distal end tip is formed easily and more securely.

### BRIEF DESCRIPTION OF THE DRAWINGS

FIG. 1 is a front view illustrating a first embodiment of a catheter of the present invention;
FIG. 2 is a section view illustrating a distal end of the catheter of FIG. 1;
FIG. 3 is a section view illustrating a distal end of a second embodiment of the catheter of the present invention;
FIG. 4 is a front view illustrating a third embodiment of the catheter of the present invention;
FIG. 5 is a section view illustrating a distal end of the catheter of FIG. 4; and
FIG. 6 is a section view illustrating a distal end of a fourth embodiment of the catheter of the present invention.

### DETAILED DESCRIPTION OF THE PREFERRED EMBODIMENTS

In the following, preferred embodiments of the invention will be described with reference to the enclosed drawings. However, the invention is not limited thereto. In the drawings, the left side corresponds to the distal end side to be inserted into a body, while the right side corresponds to the proximal end side to be operated by a technician such as a physician and is exposed outside the body without being inserted into the body. Note that the sizes of components in the drawings are exaggerated to facilitate understanding.

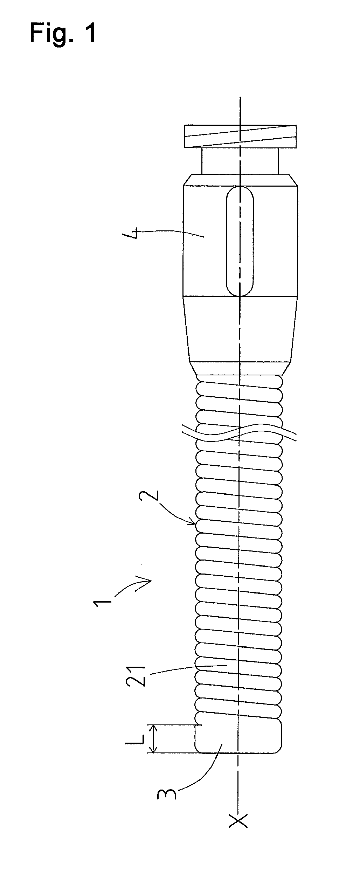

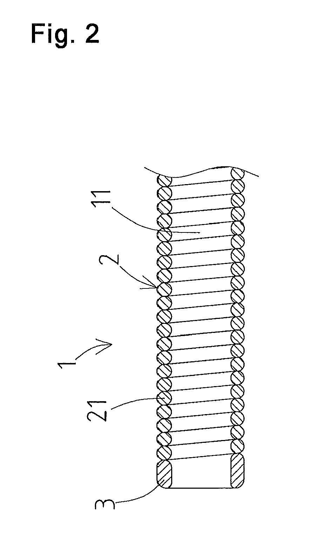

FIG. 1 and FIG. 2 are diagrams illustrating a first embodiment of the present invention. FIG. 1 is a front view illustrating a catheter of a first embodiment, and FIG. 2 is a section view illustrating a distal end of the catheter of the first embodiment of FIG. 1.

The catheter 1 of the first embodiment includes a hollow cylindrical main body 2, and a distal end tip 3 formed at the distal end of the cylindrical main body 2. Moreover, a connector 4 is normally connected to a proximal end of the hollow cylindrical main body 2 that is not inserted into a body.

The catheter 1 is inserted into a body lumen of a blood vessel and the like for use. In general, the catheter 1 is 600 mm to 1500 mm in length and 0.5 mm to 1. 5 mm in diameter, and includes a lumen 11 passing through the catheter 1 from the proximal endtothedistalendthereof. A guide wire, another catheter or the like, which are used together with the catheter 1 in treatment, may be inserted into the lumen 11.

The cylindrical main body 2 is a hollow long body extending in the longitudinal direction of the catheter 1. In FIG. 1, the cylindrical main body 2 extends to cover the substantially whole length of the catheter 1. However, it is sufficient that at least the distal end side of the catheter 1 is constituted by the cylindrical main body 2. Thus, the distal end side of the catheter 1 maybe constituted by the cylindrical main body 2, and another cylindrical body may be connected to the proximal end side of the cylindrical main body 2, for example.

The cylindrical main body 2 is formed by winding metallic wire 21 into a helical coil structure around a central axis X in the longitudinal direction. In FIG. 1, the cylindrical main body 2 is formed by winding the metallic wire 21 into a left-handed helical coil structure toward the distal end. However, the metallic wire 21 may be wound into a right-handed helical coil structure toward the distal end. Moreover, it is preferable that the metallic wire 21 is densely wound without space between wound parts of the metallic wire 21 which are adjacent with each other, as illustrated in FIG. 1. However, as long as the form of the cylindrical main body 2 is maintained, the metallic wire 21 may be roughly wound with space between wound parts of the metallic wire 21 which are adjacent with each other, or a densely wound section and a roughly wound section may be combined.

Furthermore, the cylindrical main body 2 does not need to be formed by winding a metallic wire into a helical coil structure unless the function as the cylindrical main body constituting the catheter is deteriorated. For example, the cylindrical main body 2 may be formed by braiding metallic wire.

The metallic wire 21 is a long linear body formed of metal. The metal forming the metallic wire 21 may be stainless steel, platinum, tungsten, and the like, for example. As the form of the metallic wire 21, the section thereof may be circular, oval, or rectangular, for example.

The metallic wire 21 forming the cylindrical main body 2 may be a single piece or a plurality of pieces. The metallic wire 21 may be all formed of the same material, or may be formed of the combination of metallic wire of different materials. For example, the cylindrical main body 2 may be formed of only metallic wire made of stainless steel, or may be formed of a combination of radiotransparent metallic wire made of stainless steel and radiopaque metallic wire made of platinum or tungsten.

The cylindrical main body 2 of the first embodiment of FIG. 1 is formed of stainless steel, and formed by densely winding a single piece of metallic wire 21 having a circular section into a helical coil structure without space between wound parts of the metallic wire 21 which are adjacent with each other.

The distal end tip 3 is formed at the distal end of the cylindrical main body 2 by melting and then solidifying the metallic wire 21 forming the cylindrical main body 2.

The distal end tip 3 is not a separate member from the cylindrical main body 2 but is formed by melting the metallic wire 21 forming the cylindrical main body 2. Thus, the distal end tip 3 is formed of the material same as the metallic wire 21, and the distal end tip 3 and the metallic wire 21 are not connected to each other on their surfaces as separate members but are originally the same member. Therefore, the distal end tip 3 and the cylindrical main body 2 are excellent in integrity therebetween. This effectively prevents a rupture at a border between the distal end tip 3 and the cylindrical main body 2.

The distal end tip 3 is formed at the distal end of the cylindrical main body 2 as not to close the lumen 11. The distal end tip 3 is formed from the most distal end of the catheter 1 toward the proximal end side in the longitudinal direction, and the length L of the distal end tip 3 is preferable 0.2 mm as a lower limit and 10 mm as an upper limit. When the length of the distal end tip 3 is in such a range, the distal end of the catheter 1 has excellent passing performance into a occluded portion, a stenosed portion, and the like, and excellent rigidity, and the flexibility at the distal end of the catheter 1 is not deteriorated. When a separate member is attached, like in a conventional manner, the length of a distal end tip is made longer to secure workability and connection strength, for example. By contrast, the distal end tip 3 of the first embodiment is formed by melting the metallic wire 21. Thus, the distal end tip 3 does not become longer than necessary, and the flexibility at the distal end of the catheter 1 is not deteriorated.

Examples of methods of forming the distal end tip 3 include a method in which the metallic wire 21 is wound around a mandrel to form the cylindrical main body 2 around the mandrel, the distal end tip 3 is formed by heating the distal end of the cylindrical main body 2 wound around the mandrel by laser irradiation, for example, to melt the metallic wire 21 in a desired range, the melted metallic wire 21 at the distal end is cooled naturally or forcedly to solidify the melted metallic wire 21 and form the distal end tip 3, and the mandrel is finally removed. The form of the distal end tip 3 may be adjusted by grinding or polishing, for example.

When the heating is performed by laser irradiation, as described above, it is possible to easily adjust an area of the metallic wire 21 to be melted. Moreover, when the mandrel is used to form the distal end tip 3, the lumen 11 of the catheter 1 is not closed by melted metal of the metallic wire 21, and thus the distal end tip 3 is formed securely.

As the connector 4, a connector conventionally connected to proximal ends of various catheters is applied as needed. A guide wire, another catheter, or the like, which are used together with the catheter 1 in treatment, are introduced to the lumen 11 through an opening of the connector 4.

Each of the cylindrical main body 2 and the distal end tip 3 maybe coated with resin on the outer surface, the inner surface, or both of the outer surface and the inner surface thereof. The coating may be performed entirely or partially. When the coating is performed, the distal end tip 3 is formed before coating. In this way, the integrity between the cylindrical main body 2 and the distal end tip 3 is not deteriorated by the resin forming the coating.

The resin forming the coating may be polytetrafluoroethylene, polyamide, or polyvinylpyrrolidone, for example.

A guide wire preliminarily introduced to a lumen of a blood vessel and the like is inserted into the lumen 11 of the catheter 1, and the catheter 1 is introduced to the lumen of the blood vessel and the like along the guide wire and the like, and then the catheter 1 is passed through the lumen of the blood vessel and the like until reaching a target region such as a occluded portion or a stenosed portion. After reaching the target region, the catheter 1 is rotated or moved forward or backward while the distal end thereof, that is the distal end tip 3, is in contact with the occluded portion, the stenosed portion and the like, so that the distal end tip 3 enters into the occluded portion, a stenosed portion and the like. Furthermore, the catheter 1 is repeatedly rotated or moved forward or backward so that the catheter 1 passes through the occluded portion, the stenosed portion and the like. In this manner, the occluded portion and the stenosed portion are penetrated, whereby the functions of the blood vessel and the like are recovered. If necessary, a device such as a balloon catheter or a stent, which is used together with the catheter 1, is introduced to the target region through the lumen 11 for treatment. Thereafter, the catheter 1 is taken out from the body.

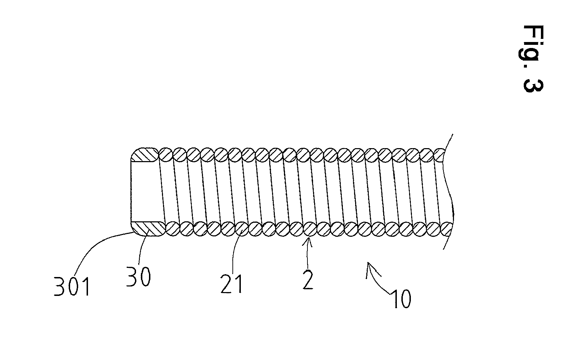

FIG. 3 is a section view illustrating a distal end of a catheter of the second embodiment of the invention.

A catheter 10 of the second embodiment has the same structure as the first embodiment except that a configuration of a distal end tip 30 is different. Therefore, the same components are represented with the same symbols in the drawings.

The catheter 10 of the second embodiment includes the hollow cylindrical main body 2 same as the first embodiment, and the distal end tip 30 is formed at the distal end of the cylindrical main body 2.

The distal end tip 30 is formed at the distal end of the cylindrical main body 2 by melting and then solidifying metallic wire 21 forming the cylindrical main body 2, similarly to the distal end tip 3 of the first embodiment.

The distal end tip 30 has a tapered surface 301 formed by grinding or polishing, for example, the outer peripheral surface of the distal end side thereof. With the tapered surface 301, the outer diameter of the distal end tip 30 is reduced toward the most distal end, which further improves passing performance of the distal end tip 30 into a occluded portion, a stenosed portion, and the like.

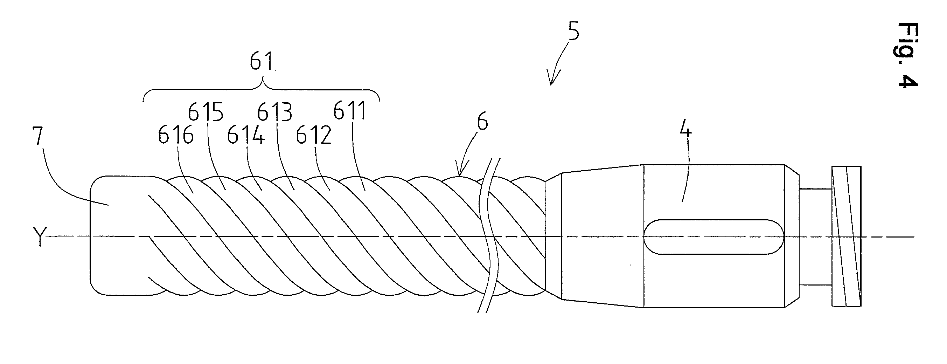

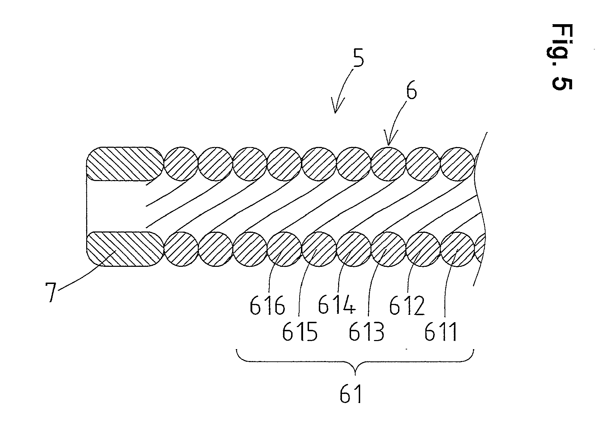

FIG. 4 and FIG. 5 are diagrams illustrating a third embodiment of the invention. FIG. 4 is a front view illustrating a catheter of the third embodiment, and FIG. 5 is a section view illustrating a distal end of the catheter of the third embodiment of FIG. 4.

A catheter 5 of the third embodiment includes a hollow cylindrical main body 6, and a distal end tip 7 is formed at the distal end of the cylindrical main body 6. Moreover, a connector 4 is normally connected to the proximal end of the cylindrical main body 6 that is not to be inserted into a body, similarly to the first embodiment.

The cylindrical main body 6 is the same as the cylindrical main body 2 of the first embodiment except for the number of metallic wires forming the cylindrical main body.

The cylindrical main body 6 is formed of stainless steel, and formed by densely winding six metallic wires 61 (611, 612, 613, 614, 615, 616) having a circular section into a right-handed helical coil structure toward the distal end around a central axis Y in the longitudinal direction. Note that the metallic wires 61 may be wound into a left-handed helical coil structure toward the distal end, and may have a roughly wound section.

The six metallic wires 611, 612, 613, 614, 615, and 616 are used in FIG. 4. However, the invention is not limited thereto as long as a plurality of metallic wires is used. With a plurality of the metallic wires 61, the flexibility of the obtained cylindrical main body 6 is further improved. On the other hand, when the flexibility of the cylindrical main body 6 is improved, a difference in flexibility between the cylindrical main body 6 and the hard metallic tip 7 formed of melted metallic wire is increased, and the stress is easily concentrated on the border between the cylindrical main body 6. Therefore, in the conventional catheter constituted by the metallic tip and the cylindrical main body as separate members, a rupture easily occurs between the metallic tip and the cylindrical main body. By contrast, in the third embodiment, the integrity between the metallic tip 7 and the cylindrical main body 6 is excellent, thereby preventing a rupture therebetween more effectively. The lower limit of the number of the metallic wires 61 is preferably 2, more preferably 6, and still more preferably 10. The upper limit of the number of the metallic wires 61 is preferably 30, and more preferably 20. The most preferable number of the metallic wires 61 is 10 to 20. When the number of the metallic wires 61 is in such a range, the above-described action effect is exerted more securely.

The distal end tip 7 is formed at the distal end of the cylindrical main body 6 by melting and then solidifying the metallic wires 61 forming the cylindrical main body 6, similarly to the method for the distal end tip 3 of the first embodiment.

Similarly to the first embodiment, the distal end tip 7 is excellent in integrity with the cylindrical main body 6, thus effectively preventing a rupture at the border between the distal end tip 7 and the cylindrical main body 6. In addition, the cylindrical main body 6 is formed of a plurality of the metallic wires 61, whereby the number of the ends of the metallic wires 61 that are positioned at the most distal end of the cylindrical main body 6 is increased by the number used. Therefore, the number of the metallic wires 61 joined each other by melting during formation of the distal end tip 7 is increased, and the distal end tip 7 is formed easily and more securely.

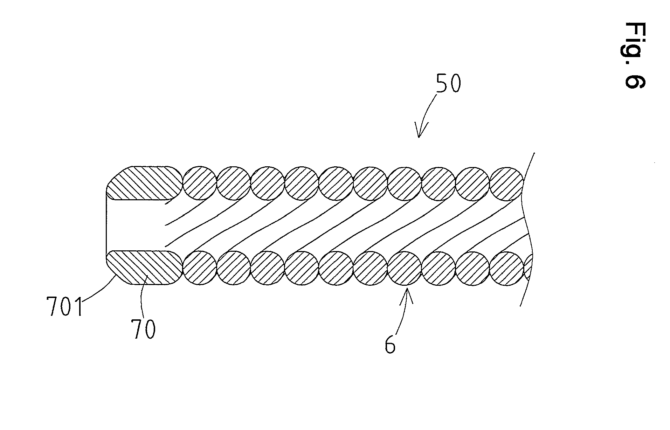

FIG. 6 is a section view illustrating a distal end of a catheter of a fourth embodiment of the present invention.

A catheter 50 of the fourth embodiment has the same structure as the third embodiment except that a configuration of a distal end tip 70 is different. Therefore, the same components are represented with the same symbols in the drawings.

The catheter 50 of the fourth embodiment includes the hollow cylindrical main body 6 same as the third embodiment, and the distal end tip 70 is formed at the distal end of the cylindrical main body 6.

The distal end tip 70 is formed at the distal end of the cylindrical main body 6 by melting and then solidifying the metallic wire 61 forming the cylindrical main body 6, similarly to the distal end tip 7 of the third embodiment.

The distal end tip 70 has a tapered surface 701 formed by grinding or polishing, for example, the outer peripheral surface of the distal end side thereof. With the tapered surface 701, the outer diameter of the distal end tip 30 is reduced toward the most distal end, which further improves passing performance of the distal end tip 70 into a occluded portion, a stenosed portion, and the like.

Moreover, the distal end tip 70 is formed longer than the distal end tip 7 of the third embodiment. The length of the distal end tip 70 may be appropriately adjusted in a range where the flexibility of the distal end of the catheter is not deteriorated. When the distal end tip 70 is formed to be long, the tapered surface 701 is formed easily, whereby it is possible to securely improve passing performance of the distal end tip 70 into a occluded portion, a stenosed portion, and the like.

### REFERENCE SIGNS LIST

1, 10, 5, 50 ... Catheter
11 ... Lumen
2, 6 ... Cylindrical main body
21, 61, 611, 612, 613, 614, 615, 616 ... Metallic wire
3, 30, 7, 70 ... Distal end tip
301, 701 ... Tapered surface
4 ... Connector

## Claims

1. A catheter (1, 10, 5, 50), comprising:
a hollow cylindrical main body (2, 6) that is formed of at least one metallic wire (21, 61); and
a hollow distal end tip (3, 30, 7, 70) that is formed at a distal end of the cylindrical main body (2, 6);
**characterized in that** the hollow distal end tip (3, 30, 7, 70) is formed by melting a distal end of the metallic wire (21, 61) of the cylindrical main body.

2. The catheter (1, 10, 5, 50) according to claim 1, **characterized in that** the cylindrical main body (2, 6) is formed by winding the metallic wire (21, 61) into a helical coil structure around a central axis (X, Y) in a longitudinal direction.

3. The catheter (5, 50) according to claim 2, **characterized in that** the cylindrical main body (6) is formed by winding a plurality of the metallic wires (611, 612, 613, 614, 615, 616) around the central axis (Y) in the longitudinal direction.
